(19) Europäisches Patentamt
European Patent Office
Office européen des brevets

(11) **EP 1 857 047 B1**

(12) **EUROPEAN PATENT SPECIFICATION**

(45) Date of publication and mention
of the grant of the patent:
**09.07.2008   Bulletin 2008/28**

(51) Int Cl.:
***A61B 5/024*** *(2006.01)*

(21) Application number: **06010245.6**

(22) Date of filing: **18.05.2006**

(54) **Method for judging irregular heartbeat**

Verfahren zur Beurteilung von unregelmässigen Herzschlägen

Méthode pour évaluer les battements de coeur irréguliers

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**21.11.2007   Bulletin 2007/47**

(73) Proprietor: **Rossmax International LTD.**
**Taipei, 114 (TW)**

(72) Inventor: **Hung, Ching-Hsi**
**Tapei 114, Taiwan (TW)**

(74) Representative: **Urner, Peter**
**TER MEER STEINMEISTER & PARTNER GbR**
**Patentanwälte**
**Mauerkircherstrasse 45**
**81679 München (DE)**

(56) References cited:
**WO-A2-20/05110215       US-A1- 2003 023 178**
**US-A1- 2006 094 967**

**Description**

1. Field of the Invention

The present invent relates to a method for judging irregular heartbeat, especially to a method for judging irregular heartbeat with higher accuracy.

2. Description of Prior Art

More than 90% of the patients of sudden death are reported to be caused by Ventricular Tachycardia (VT) or Ventricular Fibrillation (VF). Therefore, the detection of irregular heartbeat is important for preventing heart disease.

The current technology for measurement of irregular heartbeat uses Holter ambulatory ECG and Electrocardiogram, ECG. However, those services are generally conducted in professional medical institution, which is expensive for user. Therefore, the function of irregular heartbeat measurement is integrated into certain electronic sphygmomanometer for user to measure blood pressure and heart beat.

Taiwan Patent No. 522004 with title "Method and apparatus for non-invading measurement of blood pressure and heart beat" provides a method for measuring blood pressure by artery pulse. The inter-pulse duration value is measured with blood pressure and recorded in storage unit. The stored inter-pulse duration value is compared with a predetermined reference. When the stored inter-pulse duration value exceeds a predetermined reference or has a specific pattern, the patient can be judged to have irregular heartbeat.

Moreover, Taiwan Patent No. 555545 with title "System for judging irregular heartbeat with Electrocardiogram" provides a medical judgment. The system takes a time record from a long-term database of a patient and a real-time measurement of electrocardiogram from the patient. The data is processed by re-sampling, noise-removing and signal shift and then compared with a pathology database. The comparison result is used for medical evaluation.

The above-mentioned prior art methods compares measured electrocardiogram signal and pulse signal with a standard condition to generated an averaged parameter. However, the patient has different heart beat characteristics. Therefore, the above-mentioned prior art methods are not applied to all users. The setting of standard condition will also influence measurement results.

US 2003/0023178 A1 is concerned with a cardiac rhythm monitoring device and discloses a method for judging irregular heart beat, wherein beat-to-beat heart rhythms, i.e. the R-R interval between individual heart beats, are detected and a screening test is performed to determine if there are indications of arrhythmia. The test looks for variants in the R-R interval that is outside the normal range using normal distribution analysis of the patients own heart rhythm to determine the normal range of variants for determining irregular heart beats. If there are multiple irregularities within a certain time frame, the patient is warned accordingly.

In particular, a average inter-pulse duration is calculated, then it is checked whether any of the measured inter-pulse duration is more than the averaged inter-pulse duration by a predetermined ratio and to accumulate a count of the inter-pulse duration being outside the normal range. If the counted number of irregular inter-pulse duration is indicative of a sustained pattern of irregularity, the result is outputted to indicate a possibility of arrhythmia.

SUMMARY OF THE INVENTION

The object underlying the present invention is to provide a method for judging irregular heart beat with higher accuracy.

This object is obtained by a method according to claim 1.

BRIEF DESCRIPTION OF THE DRAWINGS

The features of the invention believed to be novel are set fourth with particularity in the appended claims. The invention itself however may be best understood by reference to the following detailed description of the invention, which describes certain exemplary embodiments of the invention, taken in conjunction with the accompanying drawings in which:

Fig. 1 shows a flowchart of the judgment steps according to the present invention.
Fig. 2 shows another flowchart of the judgment steps according to the present invention.
Fig. 3 shows the heartbeat pattern measured by cuff.
Fig. 4 shows the heartbeat pattern measured by cuff.
Fig. 5 shows the flowchart of judgment procedure.

## DETAILED DESCRIPTION OF THE INVENTION

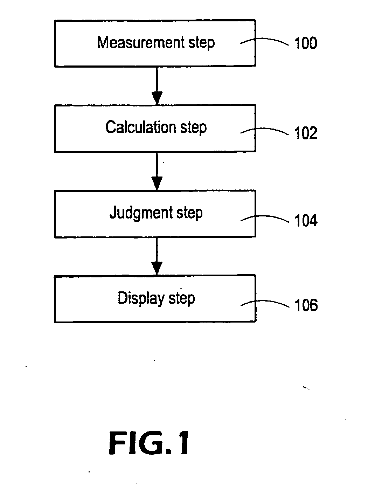

With reference to Figs. 1 to 3, the method for judging irregular heartbeat according to the present invention measures the irregular heartbeat according to the data within a time period.

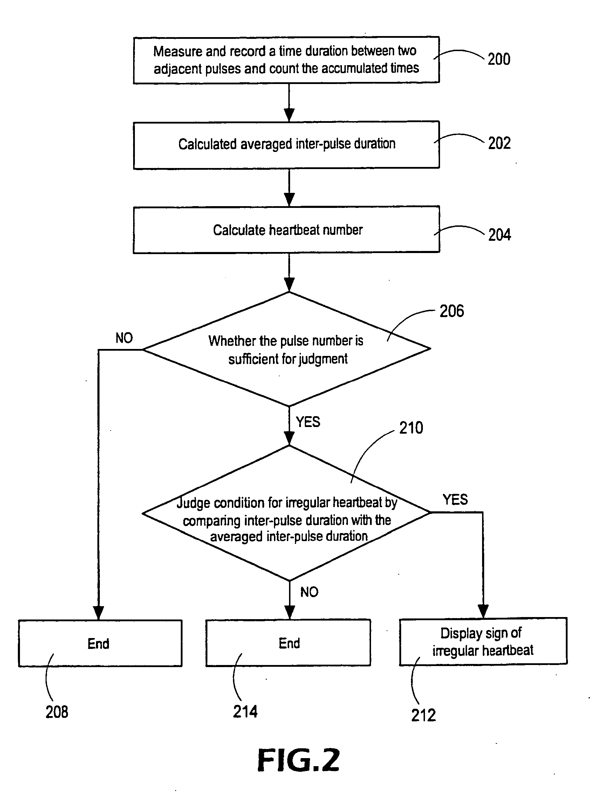

Step 100 is a measurement step, where the heart beat number is also measured when the user is measured with blood pressure. The inter-pulse duration ($\Delta T$) between two pulses and the accumulated pulse number are also recorded, as can be seen in step 200 of Fig. 2.

The inter-pulse duration ($\Delta T$) between two pulses is the time difference between two adjacent pulses.

$\Delta T1 = T2 - T1$ is the time duration difference between the first pulse and the second pulse.

$\Delta T 2 = T3 - T2$ is the time duration difference between the second pulse and the third pulse.

$\Delta T 3 = T4 - T3$ is the time duration difference between the third pulse and the fourth pulse.

$\Delta Tn = T(n+1) - Tn$ is the time duration difference between the n-th pulse and the (n+1)th pulse.

Step 102 performs a calculation step.

The averaged inter-pulse duration is calculated according to the result above, as shown in step 202 of Fig. 2. The averaged inter-pulse duration (T) = the sum of total measured inter-pulse duration divided by the total pulse number (N), as shown in formula 1.

$$T = \frac{\Delta T1 + \Delta T2 + \Delta T3 + \Delta T4 + ..... + \Delta Tn}{N} \qquad (1)$$

Afterward, the heartbeat number (H) is calculated according to the averaged inter-pulse duration (T), as shown in step 204 of Fig. 2.:

$$\text{Heartbeat (H)} = \frac{60}{\text{Average inter pulse time (T)}} (\text{times/min}) \qquad (2)$$

Step 104 performs a judgment step for the first-stage judgment. As shown in step 206 of Fig. 2, the step 206 judges whether the total number of irregular heartbeat reaches a predetermined number Z. When the number of irregular heartbeat is smaller than Z, the judgment step is finished in step S208.

Step 210 performs the second-stage judgment when the number of irregular heartbeat is judged to be larger than Z. Each of the inter-pulse durations is compared with the averaged inter-pulse duration (T). The second-stage judgment judges whether the inter-pulse duration is an abnormal inter-pulse duration, which exceeds the averaged inter-pulse duration by a predetermined ratio. The second-stage judgment further judges whether the number of the abnormal inter-pulse duration exceeds a predetermined count.

Step 106 performs a display step to show a sign of irregular heartbeat when the above-mentioned second-stage judgment for irregular heartbeat is matched, as shown in step 212 of Fig. 2. When the above-mentioned second-stage judgment for irregular heartbeat is not matched, step 212 of Fig. 2 shows a sign of no irregular heartbeat.

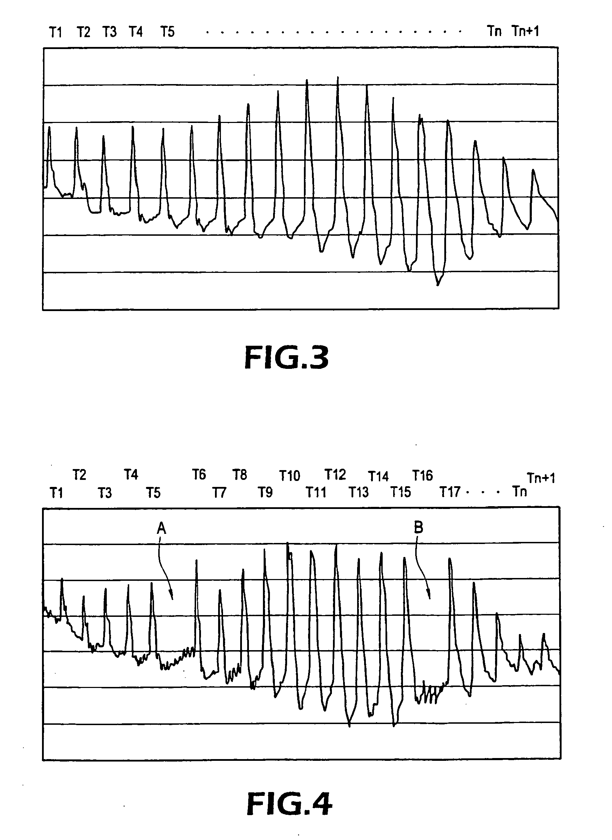

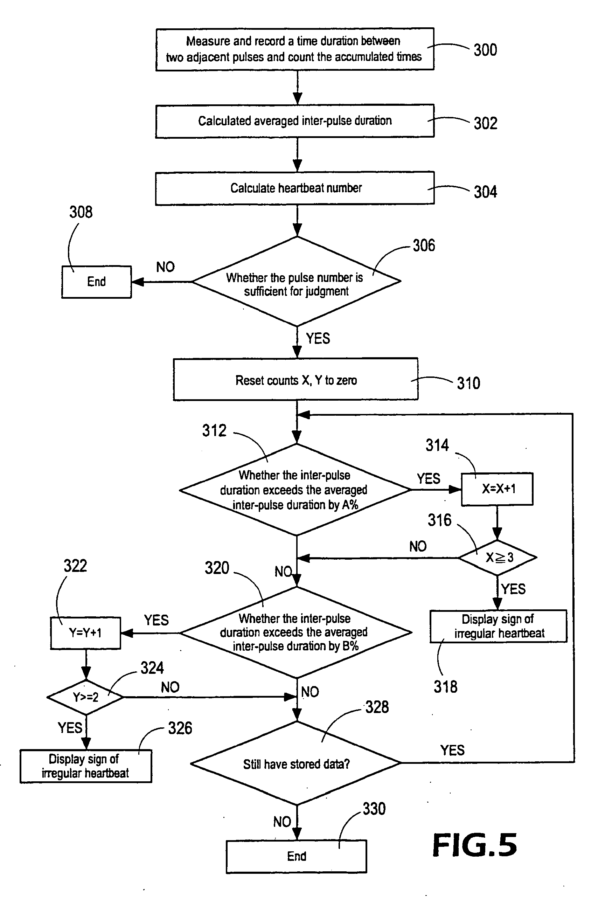

Fig. 4 shows the heartbeat pattern measured by cuff. Fig. 5 shows the flowchart of judgment procedure. In step 300, the inter-pulse duration ($\Delta T$) for each pulse is recorded and total pulse numbers are counted. As shown in Fig. 4, the inter-pulse durations A and B have different values with others. Therefore, the inter-pulse time A and B are judged to be inter-pulse duration signal of irregular heartbeat.

Step 302 performs the calculation step to calculate the averaged inter-pulse duration T, where the averaged inter-pulse duration T is the sum of the total inter-pulse duration divided by the total pulse number N, according to formula (1). Step 304 calciulates the heartbeat number (H) according to formula (2).

After the pulse duration and the heartbeat number are measured, judgment steps are then performed. A first-stage judgment is first performed, step 306 judges whether the total number of inter-pulse duration is larger than a threshold Z, such as 16 times. When the total number of the inter-pulse duration is smaller than the threshold Z, the procedure is ended at step 308. When the total number of the inter-pulse duration is larger than a threshold Z, a second-stage judgment is performed.

In the second-stage judgment step, there are two conditions for judging the irregular heartbeats. In the first condition, the irregular heartbeat is ensured when the number of inter-pulse duration, which is more than averaged inter-pulse duration by A%, is more than or equal to 3. In the second condition, the irregular heartbeat is ensured when the number of abnormal inter-pulse duration, which is more than averaged inter-pulse duration by B%, is more than or equal to 2. The irregular heartbeat is ensured when either condition is matched.

In the step 310, the counts for the abnormal inter-pulse duration X, Y are reset to zero. Step 312 checks whether an inter-pulse duration is more than the averaged inter-pulse duration by A%. If true, step 314 is performed to increase the count X by one and step 316 is performed to judge whether the count X is more than or equal to 3. If true, step 318 indicates that irregular heartbeat is ensured.

If the first condition is not met, step 320 starts the second condition by judging whether an inter-pulse duration is more than the averaged inter-pulse duration by B%. If true, step 322 is performed to increase the count of Y and step 324 is performed to judge whether the count Y is more than or equal to 2. If true, step 326 indicates that irregular heartbeat is ensured. Step 328 judges whether there is additional heartbeat data to be processed. If true, the procedure is back to step 312, else the procedure is ended at step 330.

In the above-mentioned method, an averaged inter-pulse duration is first obtained by performing measurement for a time period. The averaged inter-pulse duration is then compared with each of the measured inter-pulse durations. In other word, the reference parameter (the averaged inter-pulse duration) and the measured inter-pulse durations are from the same user. Therefore, the method for judging irregular heartbeat according to the present invention can be adapted to different user without the limitation imposed by preset parameter.

Although the present invention has been described with reference to the preferred embodiment thereof, it will be understood that the invention is not limited to the details thereof. Various substitutions and modifications have suggested in the foregoing description, and other will occur to those of ordinary skill in the art. Therefore, all such substitutions and modifications are intended to be embraced within the scope of the invention as defined in the appended claims.

## Claims

1. A method for judging irregular heartbeat, comprising:

    - performing a measurement step (300) to measure a plurality of heartbeat pulses within a time period for a user, and to measure inter-pulse duration (AT) and total pulse numbers (N), wherein the inter-pulse duration ($\Delta T$) is a time difference between two adjacent pulses;
    - performing a calculation step (302) to calculate an averaged inter-pulse duration (T), wherein the averaged inter-pulse duration (T) is calculated by dividing the sum of all inter-pulse durations ($\Delta T$) by the total pulse number (N);
    - performing a checking step (312, 320) to check whether any of the measured inter-pulse durations ($\Delta T$) is more than the averaged inter-pulse duration (T) by a predetermined ratio (A%, B%) and to accumulate (314, 322) a first count (X, Y) of the inter-pulse duration (($\Delta T$) exceeding the predetermined ratio (A%, B%);
    - performing a second-stage judgment step (316, 324) to determine an irregular heart-beat condition if the count (X, Y) is larger than a first predetermined threshold count (3, 2); and
    - performing a display step (318, 326) to show a sign of irregular heartbeat when the irregular heartbeat condition is matched, or show a sign of no irregular heartbeat when the irregular heartbeat condition is not matched

    **characterized in that**
    the checking step further comprises checking whether any of the measured inter-pulse durations are more than the averaged inter-pulse durations (T) by a second predetermined ratio different from the first predetermined ratio and accumulating a second count of the inter-pulse durations exceeding the second predetermined ratio, and the second-stage judgement step further comprises determining an irregular heartbeat condition if the count (X,Y) is larger than a second predetermined threshold count, said second predetermined threshold count being different from the first predetermined threshold count.

2. The method for judging irregular heartbeat according to claim 1, further comprising a first-stage judgment step (306) to compare the number of inter-pulse duration with a first predetermined threshold (Z) whereby the checking step (312, 320) and the judgment step are performed (316, 324), if the first-stage judgment (306) is matched.

## Patentansprüche

1. Verfahren zum Beurteilen eines unregelmäßigen Herzschlags, das umfasst:

    - Ausführen eines Messschrittes (300), um für einen Benutzer mehrere Herzschlagpulse innerhalb einer Zeitdauer zu messen und um die Zwischenpulsdauer ($\Delta T$) und die Gesamtpulsanzahl (N) zu messen, wobei die Zwischenpulsdauer ($\Delta T$) eine Zeitdifferenz zwischen zwei benachbarten Pulsen ist;

- Ausführen eines Berechnungsschrittes (302), um eine durchschnittliche Zwischenpulsdauer (T) zu berechnen, wobei die durchschnittliche Zwischenpulsdauer (T) durch Dividieren der Summe aus allen Zwischenpulsdauern (ΔT) durch die Gesamtpulsanzahl (N) berechnet wird;
- Ausführen eines Prüfschrittes (312, 320), um zu prüfen, ob irgendeine der gemessenen Zwischenpulsdauern (ΔT) um ein vorgegebenes Verhältnis (A %, B %) höher als die durchschnittliche Zwischenpulsdauer (T) ist, und um einen ersten Zählwert (X, Y) der Zwischenpulsdauer (ΔT), die das vorgegebene Verhältnis (A %, B %) übersteigt, zu akkumulieren (314, 322);
- Ausführen eines Beurteilungsschrittes (316, 324) der zweiten Stufe, um einen Zustand eines unregelmäßigen Herzschlags zu bestimmen, falls der Zählwert (X, Y) größer als ein erster vorgegebener Schwellenzählwert (3, 2) ist; und
- Ausführen eines Anzeigeschrittes (318, 326), um ein Symbol für einen unregelmäßigen Herzschlag anzuzeigen, wenn der Zustand mit unregelmäßigem Herzschlag vorhanden ist, oder um ein Symbol für keinen unregelmäßigen Herzschlag anzuzeigen, wenn der Zustand eines unregelmäßigen Herzschlags nicht besteht,

**dadurch gekennzeichnet, dass**
der Prüfschritt ferner das Prüfen umfasst, ob irgendeine der gemessenen Zwischenpulsdauern um ein zweites vorgegebenes Verhältnis, das von dem ersten vorgegebenen Verhältnis verschieden ist, größer als die durchschnittliche Zwischenpulsdauer (T) ist, und um einen zweiten Zählwert der Zwischenpulsdauern, die das zweite vorgegebene Verhältnis übersteigen, zu akkumulieren, und der Beurteilungsschritt der zweiten Stufe ferner das Bestimmen eines Zustandes eines unregelmäßigen Herzschlags umfasst, falls der Zählwert (X, Y) größer als ein zweiter vorgegebener Schwellenzählwert ist, wobei der zweite vorgegebene Schwellenzählwert von dem ersten vorgegebenen Schwellenzählwert verschieden ist.

2. Verfahren zum Beurteilen eines unregelmäßigen Herzschlags nach Anspruch 1, das ferner einen Beurteilungsschritt (306) einer ersten Stufe umfasst, um die Anzahl von Zwischenpulsdauern mit einem ersten vorgegebenen Schwellenwert (Z) zu vergleichen, wobei der Prüfschritt (312, 320) und der Beurteilungsschritt (316, 324) ausgeführt werden, wenn die Beurteilung (306) erster Stufe eine Übereinstimmung ergibt.

**Revendications**

1. Procédé pour juger qu'on est en présence de battements cardiaques irréguliers, comprenant les étapes suivantes :

- exécution d'une étape de mesure (300) pour mesurer une pluralité d'impulsions de battements cardiaques pendant une période temporelle pour un utilisateur, et pour mesurer une durée inter-impulsions (ΔT) et un nombre total d'impulsions (N), la durée inter-impulsions (ΔT) étant une différence temporelle entre deux impulsions adjacentes ;
- exécution d'une étape de calcul (302) pour calculer une durée inter-impulsions moyenne (T), de sorte que la durée inter-impulsions moyenne (T) est calculée en divisant la somme de toutes les durées inter-impulsions (ΔT) par le nombre total d'impulsions (N) ;
- exécuter une étape de vérification (312, 320) pour vérifier si l'une quelconque des durées inter-impulsions mesurées (ΔT) est supérieure à la durée inter-impulsions moyenne (T) par un rapport prédéterminé (A %, B %) et pour accumuler (314, 322) un décompte final (X, Y) de la durée inter-impulsions (ΔT) qui dépasse le rapport prédéterminé (A%,B%);
- exécuter une étape de jugement de second stade (316, 326) pour déterminer une condition de battements cardiaques irréguliers si le décompte (X, Y) est supérieur à un décompte seuil prédéterminé final (3, 2) ; et
- exécuter une étape d'affichage (318, 326) pour montrer un signe de battements cardiaques irréguliers quand la condition de battements cardiaques irréguliers est établie, ou pour montrer un signe qu'il n'y a pas de battements cardiaques irréguliers lorsque la condition de battements cardiaques irréguliers n'est pas établie,

**caractérisé en ce que**
l'étape de vérification comprend en outre de vérifier si l'une quelconque des durées inter-impulsions mesurées est supérieure à la durée inter-impulsions moyenne (T) d'un second rapport prédéterminé différent du premier rapport prédéterminé, et accumuler un second décompte des durées inter-impulsions qui dépassent le second rapport prédéterminé, et l'étape de jugement de second stade comprend en outre de déterminer une condition de battements cardiaques irréguliers si le décompte (X, Y) est supérieur à un second décompte seuil prédéterminé, ledit second décompte seuil prédéterminé étant différent du premier décompte seuil prédéterminé.

2. Procédé pour juger qu'on est en présence de battements cardiaques irréguliers, selon la revendication 1, comprenant en outre une étape de jugement de premier stade (306) pour comparer le nombre de durées inter-impulsions avec un premier seuil prédéterminé (Z), suite à quoi l'étape de vérification (312, 320) et l'étape de jugement (316, 324) sont exécutées si le jugement de premier stade (306) est établi.

```
┌─────────────────────────────┐
│      Measurement step       │ ──── 100
└─────────────────────────────┘
                │
                ▼
┌─────────────────────────────┐
│      Calculation step       │ ──── 102
└─────────────────────────────┘
                │
                ▼
┌─────────────────────────────┐
│       Judgment step         │ ──── 104
└─────────────────────────────┘
                │
                ▼
┌─────────────────────────────┐
│        Display step         │ ──── 106
└─────────────────────────────┘
```

# FIG. 1

```
┌─────────────────────────────────────────────┐
│ Measure and record a time duration between two │──── 200
│ adjacent pulses and count the accumulated times │
└─────────────────────────────────────────────┘
                      │
                      ▼
┌─────────────────────────────────────────────┐
│      Calculated averaged inter-pulse duration   │──── 202
└─────────────────────────────────────────────┘
                      │
                      ▼
┌─────────────────────────────────────────────┐
│           Calculate heartbeat number            │──── 204
└─────────────────────────────────────────────┘
                      │
                      ▼
              ◇─────────────◇  ── 206
        NO   ╱  Whether the pulse number is  ╲
     ┌───────  sufficient for judgment  ───────
     │        ╲                         ╱
     │         ◇───────────────────────◇
     │                  │ YES
     │                  ▼                ── 210
     │         ◇─────────────────◇
     │       ╱ Judge condition for irregular heartbeat by ╲   YES
     │      ─ comparing inter-pulse duration with the  ─────────┐
     │       ╲ averaged inter-pulse duration ╱                  │
     │         ◇─────────────────◇                              │
     │                  │ NO                                    │
     ▼                  ▼                                       ▼
┌─────────┐      ┌─────────┐                          ┌──────────────────┐
│   End   │      │   End   │                          │  Display sign of  │
└─────────┘      └─────────┘                          │ irregular heartbeat│
     │                │                               └──────────────────┘
    208              214                                        │
                                                               212
```

FIG.2

T1  T2  T3  T4  T5  · · · · · · · · · · · · · · · · · · · Tn  Tn+1

# FIG.3

T2      T4          T6    T8    T10    T12    T14    T16              Tn+1
T1      T3      T5          T7      T9      T11      T13      T15      T17 · · · Tn

# FIG.4

```
┌─────────────────────────────────────────┐
│ Measure and record a time duration between│──── 300
│ two adjacent pulses and count the accumulated times│
└─────────────────────────────────────────┘
                    │
                    ▼
┌─────────────────────────────────────────┐
│ Calculated averaged inter-pulse duration │──── 302
└─────────────────────────────────────────┘
                    │
                    ▼
┌─────────────────────────────────────────┐
│ Calculate heartbeat number               │──── 304
└─────────────────────────────────────────┘
                    │
                    ▼
```

308

```
┌─────┐   NO        ◇ 306
│ End │◄─────── Whether the pulse number is
└─────┘          sufficient for judgment
                        │ YES
                        ▼
┌─────────────────────────────────────────┐
│ Reset counts X, Y to zero                │──── 310
└─────────────────────────────────────────┘
```

312

```
        ◇ Whether the inter-pulse    YES    ┌───────┐
          duration exceeds the averaged ──────►│ X=X+1 │  314
          inter-pulse duration by A%          └───────┘
                    │ NO                           │
                                              316  ◇ X≥3
                                         NO ◄──────
                                                   │ YES
```

322                320

```
┌───────┐  YES   ◇ Whether the inter-pulse
│ Y=Y+1 │◄────── duration exceeds the averaged
└───────┘        inter-pulse duration by B%
    │                    │ NO
    ▼ 324                                  ┌──────────────────┐
  ◇ Y>=2    NO                             │ Display sign of   │
            ──────────────►  NO            │ irregular heartbeat│
    │ YES                                  └──────────────────┘
    ▼ 326                                         318
┌──────────────────┐
│ Display sign of   │          ◇ Still have stored data?  ──── YES
│ irregular heartbeat│  328
└──────────────────┘              │ NO
                                  ▼ 330
                          ┌─────┐
                          │ End │
                          └─────┘
```

**FIG.5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- TW 522004
- TW 555545

- US 20030023178 A1